# DEMANDE DE BREVET EUROPEEN

(11) **EP 0 531 181 A1**
(43) Date de publication de la demande: **10.03.1993**
(21) Numéro de dépôt: 92402212.2
(22) Date de dépôt: 03.08.1992
(51) Int. Cl.: C12N 15/81, C12N 1/19, C12P 21/02

(54) **Levures recombinantes hautement stables pour la production de protéines recombinantes**

(30) Priorité: 02.08.1991 FR 9109854
(71) Demandeur: RHONE-POULENC RORER S.A., 92160 Antony (FR)
(72) Inventeur: Fleer, Reinhard, F-91440 Bures sur Yvette (FR); Fournier, Alain, F-92000 Chatenay Malabry (FR); Yeh, Patrice, F-75005 Paris (FR)
(74) Mandataire: Savina, Jacques

(57) **Abrégé**

La présente invention concerne de nouvelles levures recombinantes hautement stables en milieu complexe caractérisées en ce qu'il s'agit de levures du genre Kluyveromyces dans lesquelles un gène essentiel est non-fonctionnel, contenant un vecteur portant une copie fonctionnelle dudit gène.

## Description

La présente invention concerne le domaine des biotechnologies, et plus particulièrement celui des fermentations industrielles de microorganismes recombinés.

Encore plus particulièrement, elle concerne un couple hôte/vecteur hautement stable en milieu complexe, sa préparation, et son utilisation en fermentation industrielle.

Les progrès accomplis dans le domaine de la biologie moléculaire ont permis de modifier des microorganismes pour leur faire produire des protéines recombinantes déterminées, préférentiellement des protéines hétérologues. En particulier, de nombreuses études génétiques ont porté sur la bactérie E coli. Plus récemment, les levures telles que Saccharomyces, Kluyveromyces, Pichia, ou encore Hansenula, sont apparues comme des organismes hôtes prometteurs pour ce mode de production de protéines.

Toutefois, l'application industrielle de ces nouveaux modes de production est encore limitée, notamment par les problèmes d'efficacité d'expression des gènes dans ces microorganismes recombinés, et par la difficulté d'obtenir des cellules recombinées stables dans des conditions industrielles de fermentation. L'une des contraintes essentielles d'exploitation est en effet liée à la stabilité ségrégationnelle d'un vecteur d'expression dans l'hôte utilisé. A l'échelle industrielle, un vecteur doit posséder une grande stabilité sur au moins 25 générations successives, qui représentent approximativement le nombre de générations nécéssaire pour aller jusqu'à la fin d'un fermenteur industriel de type "fed batch" de 200 m³ ("Principles of Fermentation Technology" Stanburry et Whitaker, Pergamon Press, Oxford, 1984). La stabilité du vecteur doit être encore plus grande dans le cas d'une fermentation en continu, où elle doit atteindre au moins une centaine de générations.

Chez les bactéries, la solution la plus courante utilisée en laboratoire consiste à insérer sur le plasmide utilisé un gène de résistance à un antibiotique, qui leur confère la capacité de survivre et de pousser dans un milieu sélectif contenant ledit antibiotique. Cependant, en raison des contraintes de sécurité et des règlementations dans le domaine des biotechnologies, il est indispensable au niveau industriel de pouvoir éviter l'utilisation de gènes de résistance à des antibiotiques. Chez les levures, le procédé le plus couramment utilisé consiste à cultiver des cellules déficientes dans une voie de biosynthèse d'acides aminés (Trp, Leu, His) ou de bases piques (adénine) ou pyrimidiques (uracile) transformées avec un vecteur contenant un gène capable de complémenter cette déficience. Cependant, cette approche nécessite l'emploi de milieux dépourvus de l'acide aminé ou de la base pour lesquels la souche hôte est auxotrophe. L'emploi de tels milieux synthétiques présente de nombreux désavantages. En particulier, ces milieux sont onéreux, ce qui est incompatible avec une utilisation à l'échelle industrielle, et de plus ils conduisent à une croissance plus lente des cellules et à une biomasse moins élevée.

Une solution a été proposée pour éviter l'emploi d'un milieu synthétique ou de gènes de résistance à des antibiotiques, consistant à (i) muter dans la cellule hôte un gène essentiel à sa survie dans un milieu complexe et (ii) introduire une copie intacte dudit gène sur le plasmide d'expression utilisé. Ce système, dont le principe est de forcer la cellule hôte à conserver son plasmide, a permis d'augmenter la stabilité du couple hôte/vecteur. Ce système a en particulier été décrit chez E.coli pour le gène dapD codant pour la tétrahydropicolinate-N-succinyl transférase (EP 258 118), le gène valS dont le produit est une enzyme nécessaire à la synthèse protéique (Skogman et Nilsson, Gene 31 (1984) 117), et pour le gène ssb, dont le produit est indispensable à la réplication de l'ADN et à la survie de la cellule (Porter et al., Bio/technology vol 8 (1990) 47). Ferrari et al ont également décrit l'utilisation du gène de l'alanine racémase pour stabiliser un plasmide dans un mutant de B.subtilis dans lequel ce gène n'était pas fonctionnel (Bio/technology vol 3 (1985) 1003). La demande WO 86/01224 décrit un système de sélection comparable adapté à la levure S.cerevisiae. Ce système utilise la levure S.cerevisiae mutée dans 2 gènes impliqués dans la biosynthèse de l'uracile. Il consiste à (i) inactiver un des gènes de synthèse de l'uracile, (ii) transformer ladite cellule par un vecteur portant le gène actif, et (iii) bloquer l'autre voie métabolique par mutagénèse.
Une autre approche pour obtenir des systèmes d'expression stables en milieux complexes consiste à utiliser des vecteurs intégrés dans le génome de la cellule hôte. Toutefois, ce système ne permet d'obtenir qu'un faible nombre de copies du vecteur par cellule recombinée, et de plus, la fréquence de transformation est plus faible. Dans ces conditions, les niveaux d'expression de gènes hétérologues ne sont pas toujours satisfaisants. Une méthode permettant l'amplification d'un gène intégré dans le génome a par ailleurs été développée chez S.cerevisiae, en dirigeant l'intégration vers les gènes codant pour les protéines ribosomiques présents en multiples copies dans le génome. Cependant, ce système s'avère instable lorsque les gènes intégrés sont exprimés à des niveaux élevés, qu'il s'agisse de gènes homologues ou hétérologues.

Aujourd'hui, l'utilisation de nouvelles levures, différentes de S.cerevisiae, pour la production de protéines recombinantes nécessite le développement d'outils adaptés à ces microorganismes, pour résoudre notamment les problèmes de stabilité de vecteurs d'expression de gènes hétérologues. Plus précisément, les levures taxonomiquement apparentées au genre Kluyveromyces semblent posséder une capacité de sécrétion de protéines recombinantes particulièrement avantageuse. Ceci a été observé notamment dans le cas de la levure K.lactis, pour la production de chymosine (EP 96430), d'IL-1β, ou de sérum-albumine humaine (EP 361991). Cependant, il n'existe pas de vecteurs d'expression à copies multiples suffisamment stables chez cet organisme pour permettre son utilisation dans des procédés industriels. Il n'existe pas en particulier de vecteurs stables en milieux complexes, permettant d'envisager des procédés à grande echelle, et notamment en continu, au moyen de cet organisme. En effet, bien que certains vecteurs stables chez K.lactis aient été décrits (EP 361991), l'introduction sur ces vecteurs d'une cassette d'expression d'un gène hétérologue produit un effet déstabilisant important, et plus spécialement en condition d'induction de la production.

Il a maintenant été trouvé un moyen particulièrement efficace pour stabiliser des couples hôte/vecteur dans lesquels l'hôte est une levure du genre Kluyveromyces.

Un objet de l'invention réside donc dans un couple hôte/vecteur hautement stable en milieu complexe caractérisé en ce que l'hôte est une levure du genre Kluyveromyces dans laquelle un gène essentiel pour sa croissance sur ledit milieu est non-fonctionnel, et en ce que le vecteur porte une copie fonctionnelle dudit gène.

Au sens de la présente invention, on entend par milieu complexe tout milieu de fermentation industriel compatible avec les contraintes économiques d'une exploitation à grande échelle. Notamment, il s'agit de milieux contenant des matières premières de type industriel : extrait soluble de maïs, extrait de levure, mélasse, ou "distillers" par exemple, par opposition aux milieux synthétiques définis, et supplémentés (comme par exemple d'antibiotiques). Il est entendu toutefois que la présente invention peut également être utilisée sur des milieux synthétiques, bien que ce mode de mise en oeuvre soit moins avantageux.

Par ailleurs, il est entendu que le gène fonctionnel présent sur le vecteur peut être un gène homologue ou hétérologue.

Comme gènes essentiels à la croissance de la cellule hôte sur milieu complexe, on peut citer plus particulièrement des gènes impliqués dans le métabolisme d'une source carbonée présente dans le milieu (galactose, lactose, glucose, etc), des gènes intervenant dans la division cellulaire, dans la synthèse membranaire, dans la synthèse protéique, la réplication ou la transcription des ADN.

Plus préférentiellement, l'invention réside donc dans un couple hôte/vecteur hautement stable en milieu complexe caractérisé en ce que l'hôte est une levure du genre Kluyveromyces dans laquelle un gène impliqué dans la glycolyse est non-fonctionnel, et en ce que le vecteur porte une copie fonctionnelle dudit gène.

Il a en effet été montré que l'on peut obtenir, chez Kluyveromyces, des couples hôte/vecteur très stables en milieu complexe compatible avec une exploitation industrielle, en rendant la cellule hôte dépendante de son plasmide lorsqu'elle doit faire intervenir une étape de la glycolyse pour métaboliser les sources de carbone du milieu.

Plus préférentiellement, la présente invention concerne les couples hôte/vecteur dans lesquels l'hôte est choisi parmi les levures Kluyveromyces lactis et Kluyveromyces fragilis.

La glycolyse implique chez la levure une succession d'étapes enzymatiques et chimiques complexes conduisant à la formation de molécules d'ATP et d'éthanol. Les principales enzymes impliquées dans cette voie sont connues, et certains des gènes codant pour ces enzymes ont été identifiés et clonés : notamment, les gènes codant pour la phosphofructo kinase (Kopperschlager et al., Eur. J. Biochem. 81 (1977) 317) ; la pyruvate kinase (Aust et al., J. Biol. Chem. 253 (1978) 7508) ; la phosphoglycérate kinase (Scopes, Meth.Enzymol. 42 (1975) 134) ; la phosphoglycérate mutase (Price et al., FEBS Letters 143 (1982) 283) ; la triose phosphate isomérase (Alber et al., J. Biol. Chem. 256 (1981) 1356) ; la pyruvate décarboxylase (Gounarb et al., Biochim. Biophys. Acta 405 (1975) 492) ou encore la phosphoglucose isomérase (Noltmann, The enzymes, vol VI, Academic Press, N.Y., 271, 1972) ont été identifiés chez S. cerevisiae. Certains gènes glycolytiques ont également été clonés chez K.lactis. Il s'agit plus précisément des gènes RAG1 et RAG2 codant respectivement pour une protéine transporteur de sucre (Goffrini et al., Nucl. Acid. Res. 18 (1990) 5294) et pour une phosphoglucose isomérase (Wésolowski-Louvel, Nucl. Acid. Res. 16 (1988) 8714) ; et de gènes codant pour des alcool deshydrogénases, ADH (Saliola et al., Yeast 6 (1990) 193-204; Saliola et al., Yeast 7 (1991) 391-400).

Cependant, ces gènes ne peuvent être utilisés efficacement pour stabiliser des vecteurs d'expression dans les levures du genre Kluyveromyces. En effet, le gène RAG1 et les gènes ADH ne sont pas indispensables à l'utilisation du glucose. De plus, les premiers travaux sur Kluyveromyces ont montré que le gène RAG2, dont l'équivalent PGI est connu comme étant essentiel pour la croissance de la levure S.cerevisiae sur un milieu contenant du glucose, ne l'était pas chez Kluyveromyces (Wésolowski-Louvel précitée ; Goffrini et al., Yeast 5 (1989) 99-106). Ce résultat indiquait une différence de comportement des levures Saccharomyces et Kluyveromyces vis-à-vis de l'utilisation du glucose, qui ne permettait pas la réalisation, chez cette dernière, d'un couple hôte/vecteur stable en utilisant les gènes glycolytiques comme marqueurs de sélection.

Dans ces conditions, l'utilisation de ces gènes pour la stabilisation de vecteurs d'expression dans les levures du genre Kluyveromyces n'était ni décrite ni suggérée, ni possible.

De manière surprenante, la demanderesse a maintenant montré que certains gènes glycolytiques peuvent être nécéssaires à la croissance et/ou la survie de levures du genre Kluyveromyces dans un milieu complexe, et qu'ils peuvent permettre la réalisation de couples hôte/vecteur particulièrement stables. En particulier, des systèmes stables et efficaces peuvent être obtenus lorsque le gène glycolytique choisi est un gène unique dont le produit est indispensable pour que la levure puisse métaboliser les sources de carbone du milieu. En effet, certaines étapes de la glycolyse font intervenir des activités pouvant être codées par plusieurs gènes. C'est le cas notamment chez S.cerevisiae des activités énolase (ENO), phosphofructokinase (PFK), glycéraldéhyde-3-phosphate déshydrogénase (GAPDH) ou alcool déshydrogénase (ADH), qui sont codées par plusieurs gènes. Dans ce cas, l'inactivation d'une de ces activités enzymatiques nécessiterait l'inactivation de tous les gènes susceptibles de la coder.

Préférentiellement, dans le couple hôte/vecteur de l'invention, le gène impliqué dans la glycolyse est un gène unique dont le produit est indispensable pour que la levure puisse métaboliser les sources de carbone du milieu.

Encore plus préférentiellement, il s'agit d'un gène choisi parmi les gènes codant pour la phosphoglycérate kinase (PGK), la phosphoglycérate mutase (GPM), la pyruvate kinase (PYK), et la triose phosphate isomérase (TPI).

Le gène sélectionné peut être rendu non-fonctionnel dans la levure hôte de différentes manières. Notamment, il est possible d'utiliser des techniques de mutagénèse non spécifiques. Plus particulièrement, les levures peuvent être traitées par des agents physiques (rayons X ; Ultra-Violet, ...) ou chimiques (agents intercalants, agents alkylants ou bialkylants ...). Les levures ainsi traitées sont ensuite sélectionnées sur différents milieux, selon la mutation recherchée.

Il est également possible d'utiliser des outils de mutagénèse spécifiques, et notamment les techniques d'insertions mutationnelles sur l'ADN ou de remplacement de gènes par recombinaison homologue (Rothstein, Meth. Enzymol. 101 (1983) 202). A cet effet, 2 voies de mutagénèse sont possibles :
- remplacement du gène à déléter par un marqueur de sélection dominant de type marqueur de résistance à un antibiotique (généticine, fluomycine, etc). Cette stratégie peut être appliquée directement sur une souche sauvage ;
- remplacement du gène à déléter par une copie intacte d'un gène complémentant une auxotrophie (ura3, trp1, leu2, etc) de la souche utilisée. Cette stratégie peut être appliquée sur toute souche présentant une auxotrophie, ou sur une souche sauvage préalablement rendue auxotrophe.

Préférentiellement, dans le couple hôte/vecteur de l'invention, la copie fonctionnelle du gène essentiel présente sur le vecteur est placée sous le contrôle d'un promoteur faible. Ce mode avantageux de réalisation favorise la stabilité du couple et l'augmentation du nombre de copies du vecteur par cellule hôte, et, de ce fait, tend à augmenter le niveau d'expression d'un gène recombinant. A titre d'exemple de promoteur faible utilisable à cet effet, on peut citer notamment le promoteur bidirectionnel du gène de la toxine killer (Tanguy-Rougeau et al., Gene 91 (1990) 43) ou celui d'un gène hétérologue tel que le promoteur du gène de la phosphatase acide de S.cerevisiae en condition de répression (milieu de culture contenant du phosphate). Dans un autre mode préféré de l'invention, la copie fonctionnelle du gène essentiel présente sur le vecteur est soit entièrement dépourvue de promoteur, soit placée sous le contrôle d'un promoteur défectif, que ce soit à la suite d'une mutation du promoteur même ou à la suite de l'inactivation d'un gène impliqué dans l'activation transcriptionnelle dudit promoteur. Dans un autre mode de mise en oeuvre de l'invention, le gène essentiel présent sur le vecteur peut être un gène défectif dans certaines conditions, comme par exemple des conditions de température. Notamment, il peut s'agir d'un gène thermosensible.

Préférentiellement, dans le couple hôte/vecteur de l'invention, le vecteur comprend en outre une séquence d'ADN comportant un gène de structure au moins codant pour une protéine recherchée et des signaux permettant son expression.

Dans un mode préféré de l'invention, le gène de structure code pour une protéine d'intérêt pharmaceutique ou agroalimentaire.

A titre d'exemple, on peut citer les enzymes (tels que notamment la superoxide dismutase, la catalase, les amylases, les lipases, les amidases, la chymosine etc.), les dérivés sanguins (tels que la sérum-albumine ou des variants ou précurseurs de celle-ci, l'alpha- ou la béta-globine, le facteur VIII, le facteur IX, le facteur von Willebrand ou des domaines de celui-ci, la fibronectine, l'alpha-1 antitrypsine etc.), l'insuline et ses variants, les lymphokines [telles que les interleukines, les interférons, les facteurs de stimulation des colonies (G-CSF, GM-CSF, M-CSF...), le TNF, le TRF, les MIP, etc.], les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), les récepteurs viraux, ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.)).

Par ailleurs, de manière avantageuse, la séquence d'ADN comprend en outre des signaux permettant la sécrétion de la protéine recombinante. Ces signaux peuvent correspondre aux signaux naturels de sécrétion de la protéine considérée, mais ils peuvent également être d'une origine différente. En particulier des signaux de sécrétion dérivés de gènes de levure peuvent être utilisés, tels que ceux des gènes de la toxine killer ou de la phéromone alpha.

Préférentiellement, le gène de structure code pour la sérum-albumine humaine, ses précurseurs ou ses variants moléculaires. On entend par variant moléculaire de l'albumine les variants naturels résultant du polymorphisme de l'albumine, les dérivés structuraux possédant une activité de type albumine, les formes tronquées de l'albumine, ou toute protéine hybride à base d'albumine.

Dans un autre mode de réalisation, le ou les gènes de structure codent pour des polypeptides intervenant au niveau génétique ou biochimique dans la biosynthèse d'un métabolite. En particulier, il peut s'agir de gènes impliqués dans la biosynthèse d'acides aminés, d'antibiotiques ou de vitamines.

Généralement, les signaux permettant l'expression du gène de structure sont choisis parmi les promoteurs et les terminateurs de transcription. Il est entendu que ces signaux sont choisis en fonction du gène de structure et du résultat recherché. En particulier, il peut être préférable dans certains cas d'utiliser un promoteur régulable de manière à pouvoir découpler les phases de croissance de l'hôte et celle d'expression du gène. De même, pour des raisons de force et de compatibilité, il peut être préférable d'utiliser dans certains cas les promoteurs naturels des gènes de structure, et dans d'autres cas, des promoteurs d'origine différente.

Préférentiellement, les promoteurs utilisés sont dérivés de gènes de levure, et encore plus préférentiellement, de gènes glycolytiques de levure. D'un intérêt tout particulier sont les promoteurs dérivés de gènes glycolytiques des levures du genre Saccharomyces ou Kluyveromyces. Notamment, on peut citer les promoteurs des gènes codant pour la phosphoglycerate kinase (PGK), la Glyceraldehyde-3-phosphate déshydrogénase (GPD), les énolases (ENO), ou les alcool-déshydrogénases (ADH). On peut également citer des promoteurs dérivés de gènes fortement exprimés tels que le gène de la lactase (LAC4), de la phosphatase acide (PHO5), ou de facteurs d'élongation (TEF).

Par ailleurs, ces régions promotrices peuvent être modifiées par mutagénèse, par exemple pour ajouter des éléments supplémentaires de contrôle de la transcription, tels que notamment des régions UAS ("Upstream Activating Sequence"). A titre d'exemple, un promoteur hybride entre les promoteurs des gènes PGK et GAL1/GAL10 de S.cerevisiae donne de bons résultats.

Les couples hôte/vecteur de l'invention peuvent être utilisés dans des procédés de production de protéines recombinantes. Ils permettent ainsi la réalisation de systèmes de production particulièrement efficaces chez les levures du genre Kluyveromyces.

A cet égard, un autre objet de l'invention concerne un procédé de production d'une protéine recombinante selon lequel on cultive un couple hôte/vecteur tel que défini ci-avant comprenant le gène de structure codant pour ladite protéine sous le contrôle de signaux permettant son expression, et on récupère la protéine produite.

Un tel procédé permet la production de protéines d'intérêt pharmaceutiques ou agroalimentaires, telles qu'énoncées ci-avant. Il est particulièrement adapté, bien que non limité, à la production de sérum-albumine humaine, ses précurseurs et variants moléculaires.

Les couples hôte/vecteur de l'invention peuvent également être utilisés directement comme catalyseurs dans des réaction de bioconversion.

Un autre objet de l'invention concerne les vecteurs d'expression pour les levures du genre Kluyveromyces portant une copie fonctionnelle d'un gène essentiel à la croissance de la levure Kluyveromyces sur milieu complexe.

Plus préférentiellement, le gène essentiel est un gène intervenant dans l'une des fonctions précédemment citées. Ce gène peut être obtenu par toute méthode connue de l'homme du métier (clonage par hybridation en utilisant des sondes hétérologues, clonage par complémentation de mutants, etc).

Avantageusement, les vecteurs de l'invention sont dépourvus de toute séquence bactérienne. Il a en effet été montré qu'il est possible de transformer in vitro les levures Kluyveromyces avec de tels vecteurs. Ce système présente l'avantage de permettre l'utilisation de vecteurs plus petits, donc plus maniables et capables de recevoir des séquences d'ADN recombiné plus importantes.

Comme exemples de tels vecteurs, on peut citer notamment les vecteurs pYG1023, pYG1033 et pYG1033ΔSfiI, qui sont décrits dans les exemple.

Par rapport aux systèmes de l'art antérieur, les avantages de l'invention sont notamment:
- la stabilisation très efficace de plasmides dans les levures du genre Kluyveromyces:
- la possibilité de cultiver les cellules recombinantes sur milieu peu coûteux et facilement accessible en grandes quantités ; ou encore,
- la possibilité de détecter de manière très simple les cellules recombinées, ce qui rend inutile l'emploi de marqueurs de sélection supplémentaires. En effet, il est généralement nécessaire, pour identifier et/ou sélectionner les cellules recombinantes, d'utiliser un ou plusieurs marqueurs. Le plus souvent il s'agit de gènes conférant la résistance à des antibiotiques, tel que notamment la généticine (gène aph, ou à d'autres composés toxiques pour la cellule tels que les ions cuivre (gène CUP). Il peut également s'agir de gènes complémentant des auxotrophies de la cellule hôte (gènes URA3, TRP1, LEU2, etc). Les couples hôte/vecteur de l'invention permettent d'éviter l'emploi de tout autre marqueur de sélection.

La présente invention sera plus complètement décrite à l'aide des exemples suivants, qui doivent être considérés comme illustratifs et non limitatifs.

### LEGENDE DES FIGURES

Figure 1 : Carte de restriction du plasmide pYG600. bla = gène de la β-lactamase conférant la résistance à l'ampicilline.

Figure 2 : Stratégie de construction du plasmide pYG70. aph = gène de la 3′-aminoglycoside phosphotransférase conférant la résistance à la généticine (G418) ; prom = promoteur; IR = séquence répétée inversée ; ORI = origine de réplication ; LacZ′= gène de structure de la β-galactosidase.

Figure 3 : Stratégie de construction du plasmide pYG70-2. Pour la légende, voir figure 2. Les sites marqués d'une (*) possèdent des extrêmités compatibles avec les sites correspondants sans reconstituer, après ligation, de sites de coupure reconnus par lesdites enzymes.

Figure 4 : Séquence des oligodésoxynucléotides de synthèse A-H ayant servi à la construction des adaptateurs 1 à 3 et à la réaction de PCR pour contrôler le génotype des mutants pgk (exemple 3.2.(ii)).

Figure 5 : Stratégie de construction du plasmide pYG70-3. Pour la légende, voir figure 2. term = terminateur.

Figure 6 : Stratégie de construction du plasmide pYG1003. Pour la légende, voir figure 2.

Figure 7 : Stratégie de construction du plasmide pYG1023. Pour la légende, voir figure 2.

Figure 8 : Stratégie de construction du plasmide pYG1033. S=SmaI, H=HindIII, P=PvuII, Af=AflII, Av=AvrII.

Figure 9 : Représentation des vecteurs pYG1033 et pYG1033ΔSfiI. Pour la légende, voir figure 2.

Figure 10 : Stratégie de clonage, et modification du gène URA3 de K.lactis CBS2359.

Figure 11 : Stratégie de construction du plasmide pYG1012.

Figure 12 : Stratégie de construction du plasmide pYG1013 et représentation du fragment EcoRI-SpeI portant le gène PGK K.l. modifié.

Figure 13 : Cette figure présente la production d'albumine humaine par la levure FB05D transformée par le plasmide pYG1023, sur 200 générations de culture en milieu complexe de type industriel, et, parallèlement, la stabilité du plasmide pYG1023 dans cette levure au cours de la même période. La stabilité et la production d'albumine sont définies dans les exemples correspondants.

### TECHNIQUES GENERALES DE CLONAGE

Les méthodes classiques de biologie moléculaire telles que la centrifugation d'ADN plasmidique en gradient de chlorure de césium-bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning : a Laboratory Manual", Cold Spring Harbor Laboratory, Cold Spring Harbor, N.Y., 1986 ; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain Réaction") dans les conditions décrites par Mullis et Faloona (Meth. Enzym., 155 (1987) 335-350) et Saiki et al (Science 230 (1985) 1350-1354), en utilisant un "DNA thermal cycler" (Perkin Elmer Cetus) en suivant les recommandations du fabricant.

### EXEMPLES

### UTILISATION DU GENE PGK DE K.LACTIS COMME MARQUEUR DE SELECTION

Cet exemple décrit la réalisation d'un couple hôte/vecteur dans lequel le gène permettant d'augmenter la stabilité du plasmide à copies multiples est le gène codant pour la 3-phosphoglycérate kinase (PGK) de K.lactis.

Le couple ainsi obtenu est stable dans un milieu complexe de type industriel tel que précédemment défini.

### 1. Isolement du gène PGK de K.lactis.

Le gène PGK a été isolé chez K.lactis CBS2359 par ciblage d'une banque génomique partielle avec une sonde hétérologue corréspondant à la partie N-terminale du gène PGK de S.cerevisiae (Dobson et al., Nucl.Acid.Res. 10 (1982) 2625-2637). Plus précisément, la sonde utilisée correspond au fragment PvuI-EcoRI de 1,3 kb.

En Southern Blot (Southern et al., J.Biol.Chem. 98 (1975) 503), la sonde utilisée s'hybride notamment à une séquence d'ADN contenue dans un fragment HindIII-HindIII de 4 kb. Cette séquence a été isolée par hybridation sur colonies au moyen de la sonde précédente. Pour cela, une banque restreinte d'ADN génomique de la souche CBS2359, constituée de fragments HindllI d'une taille comprise entre 3 et 5 kb introduits au site HindIII du plasmide pUC18, a été préparée et criblée.

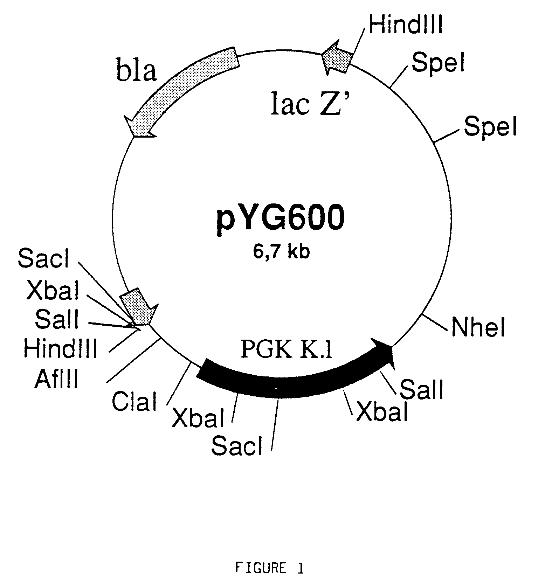

Un clone portant le plasmide pYG600 (figure 1) a ainsi été isolé. La séquence du gène PGK porté par ce plasmide a été décrite par Fournier et al. (Nucl.Acid.Res. 18 (1990) 369).

### 2. Construction de vecteurs d'expression d'une protéine recombinante portant le gène PGK de K.lactis.

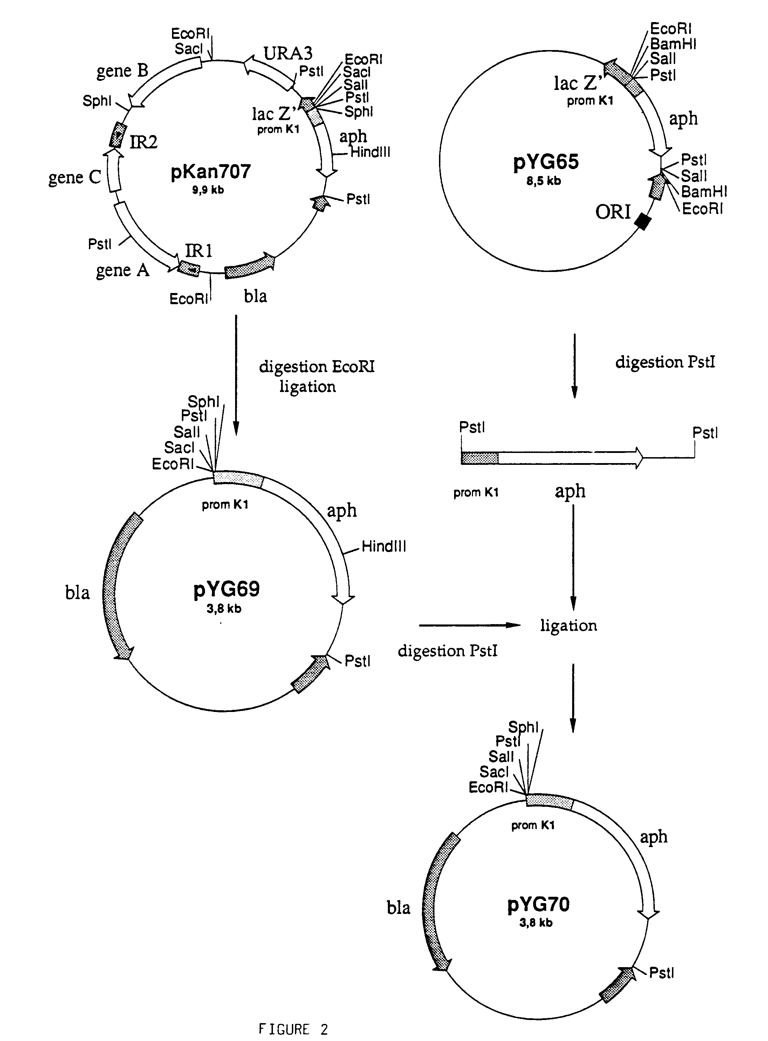

### 2.1. Construction du plasmide pYG70 (figure 2).

Le plasmide pYG70 dérive du plasmide pKan707 (voir EP361 991) par élimination des fragments EcoRI contenant le gène URA3 et la séquence du plasmide pKD1, ainsi que du site HindIII unique présent dans le gène aph, pour faciliter les étapes ultérieures de clonage. Le gène,ap: code pour l'aminoglycoside 3′-phosphotransférase (I) (Oka et al., J. Mol. Biol. 147 (1981) 217), et est utilisé comme marqueur de résistance à la généticine (G418) chez la levure. Le fragment PstI du plasmide pKan707 contenant le gène aph a été sous-cloné dans le bactériophage M13mp7. Le site HindIII présent dans ce gène a ensuite été détruit par mutagénèse dirigée selon la méthode déité par Taylor et al. (Cf techniques générales de clonage), pour générer le plasmide pYG65 (voir figure 2). L'oligodésoxynucléotide suivant a été utilisé pour réaliser cette mutagénèse :

5'-GAA ATG CAT AAG CTC TTG CCA TTC TCA CCG-3'

Cet oligodésoxynucléotide a permis de remplacer le triplet CTT codant pour la leucine 185 en CTC. Ce changement ne modifie pas la séquence protéique résultante.

Pour obtenir le plasmide pYG70, la partie contenant le réplicon bactérien de pKan707 a été isolée par digestion avec l'enzyme EcoRI et recircularisée avec la T4 DNA ligase pour former le plasmide pYG69 (figure 2). Le fragment PstI présent dans ce dernier contenant le gène aph a ensuite été remplacé par le fragment équivalent muté provenant de pYG65.

Le plasmide pYG70 ainsi obtenu contient donc :
- un réplicon et un marqueur de sélection (gène bla conférant la résistance à l'ampicilline) pour E.coli,
- un marqueur de sélection pour la levure (gène aph muté) sous contrôle du promoteur k1 de la toxine Killer.

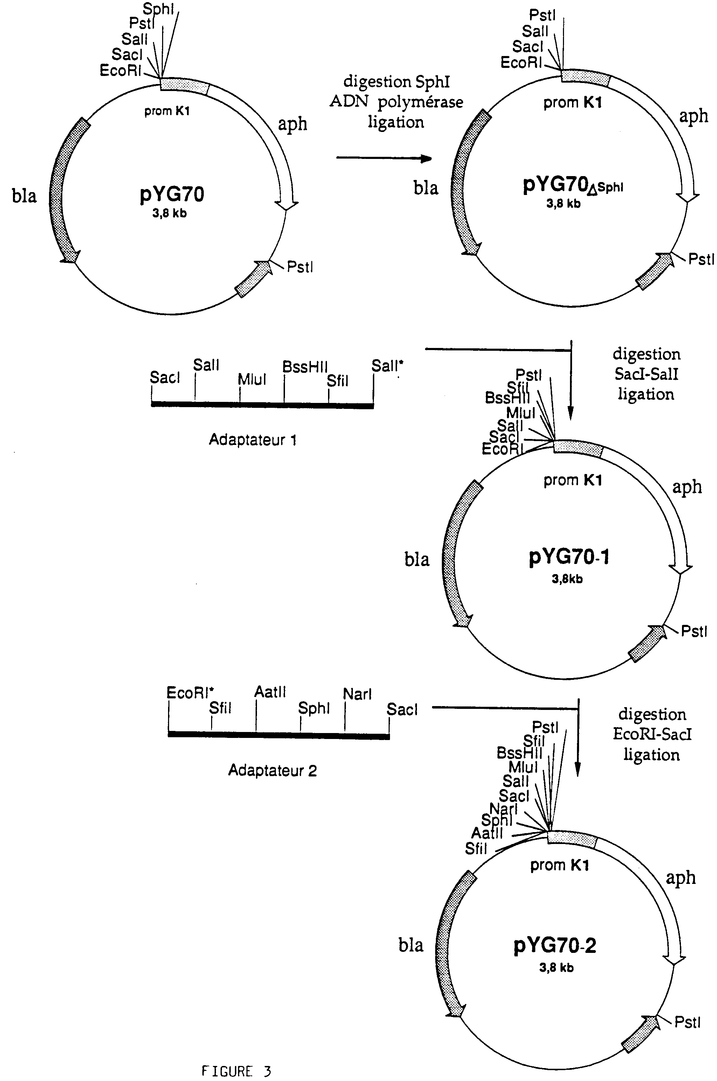

### 2.2. Modification des sites de restriction du plasmide pYG70 (figure 3).

Pour faciliter les étapes ultérieures de clonage, certains sites de restriction ont été supprimés (i) et 2 adaptateurs ont été ajoutés (ii et iii) sur le plasmide pYG70.

### (i) Elimination du site SphI.

Le plasmide pYG70 a été digéré par SphI, et les extrêmités cohésives ont ensuite été éliminées par digestion en présence d'ADN polymérase du phage T4. Après ligation en présence de ligase, le plasmide pYG70ΔSphI a été obtenu (voir figure 3).

### (ii) Insertion de l'adaptateur 1.

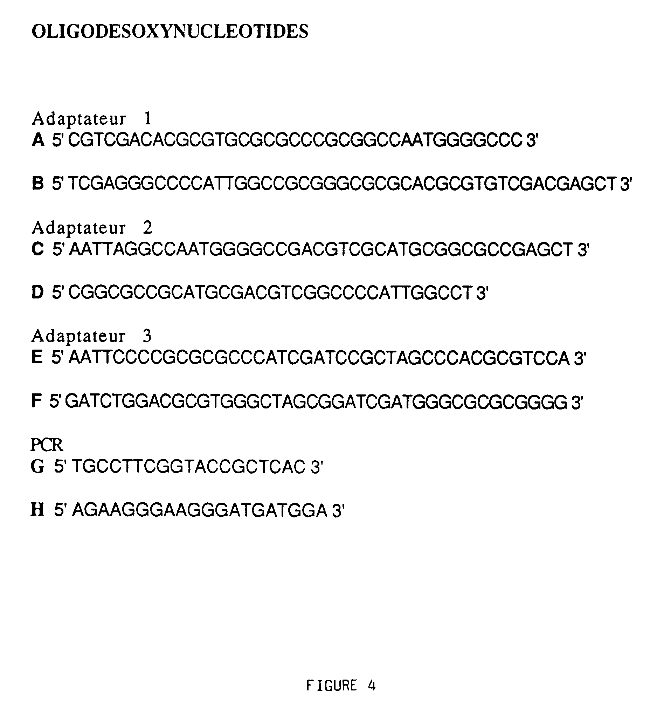

L'adaptateur 1 a été obtenu par hybridation des oligodésoxynucléotides de synthèse A et B présentés sur la figure 4. Pour cela, 2 µg de chaque oligodésoxynucléotide ont été incubés dans un tampon d'hybridation qsp 20µl (tampon Tris-HCl 30 mM pH 7,5; NaCl 30 mM; MgCl₂ 7,5 mM; ATP 0,25 mM; DTT 2 mM ; EDTA 0,2 mM), puis la température a été élevée à 80°C pendant 10 minutes, et ramenée lentement à température ambiante.

L'adaptateur ainsi obtenu contient des sites de coupure pour les enzymes suivantes : SacI ; SalI ; MluI ; BssHII et SfiI, et permet d'éliminer, lors de son introduction, le site SalI présent sur le plasmide pYG70ΔSphI. Cet adaptateur a été introduit par ligation dans le plasmide pYG70ΔSphI préalablement digéré par les enzymes SalI et SacI.

Le plasmide obtenu est appelé pYG70-1.

### (iii) Insertion de l'adaptateur 2.

L'adaptateur 2 a été réalisé en suivant le protocole décrit pour l'adaptateur 1, en utilisant les oligodésoxynucléotides C et D décrits sur la figure 4. Cet adaptateur contient des sites de coupure pour les enzymes es suivantes : SfiI ; AatII ; SphI ; NarI et SacI et permet d'éliminer, lors de son introduction, le site EcoRI présent sur le plasmide pYG70-1. Il a été introduit par ligation dans le plasmide pYG70-1 préalablement digéré par les enzymes EcoRI et SacI, pour former le plasmide pYG70-2 (figure 3).

### 2.3. Introduction d'une cassette d'expression de la sérum-albumine humaine.

La cassette d'expression de la sérum-albumine humaine utilisée comprend :
- le promoteur inductible du gène LAC4 de K.lactis,
- le gène de structure codant pour la sérum-albumine humaine (forme prépro), et
- le terminateur du gène PGK de S.cerevisiae.

Cette cassette a été isolée du plasmide pYG404 (EP361 991) sous forme d'un fragment SalI-SacI, puis introduite par ligation dans le plasmide pYG70-2 préalablement digéré par les enzymes correspondantes.

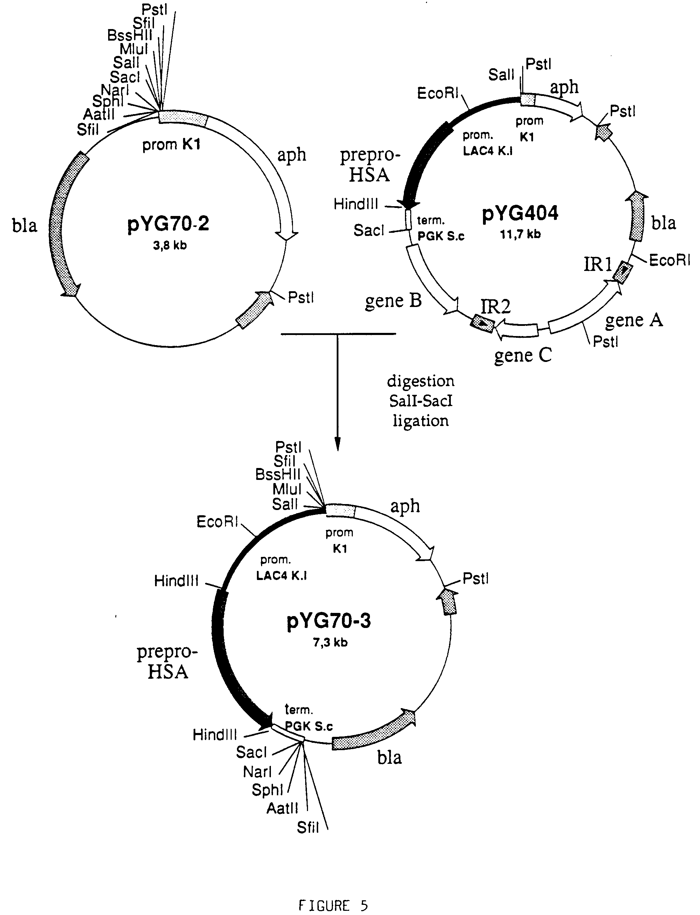

Le plasmide obtenu est appelé pYG70-3 (figure 5).

### 2.4. Insertion du gène PGK de K.lactis.

Le gène PGK K.lactis a été isolé du plasmide pYG600 (figure 1), sous-cloné dans le plasmide pYG1002 pour générer le plasmide pYG1003, puis isolé de ce dernier sous forme d'un fragment MluI-BssHII.

Le sous-clonage dans pYG1002 a permis d'obtenir le gène PGK K.lactis dépourvu de son promoteur, et sous forme d'un fragment MluI-BssHII.

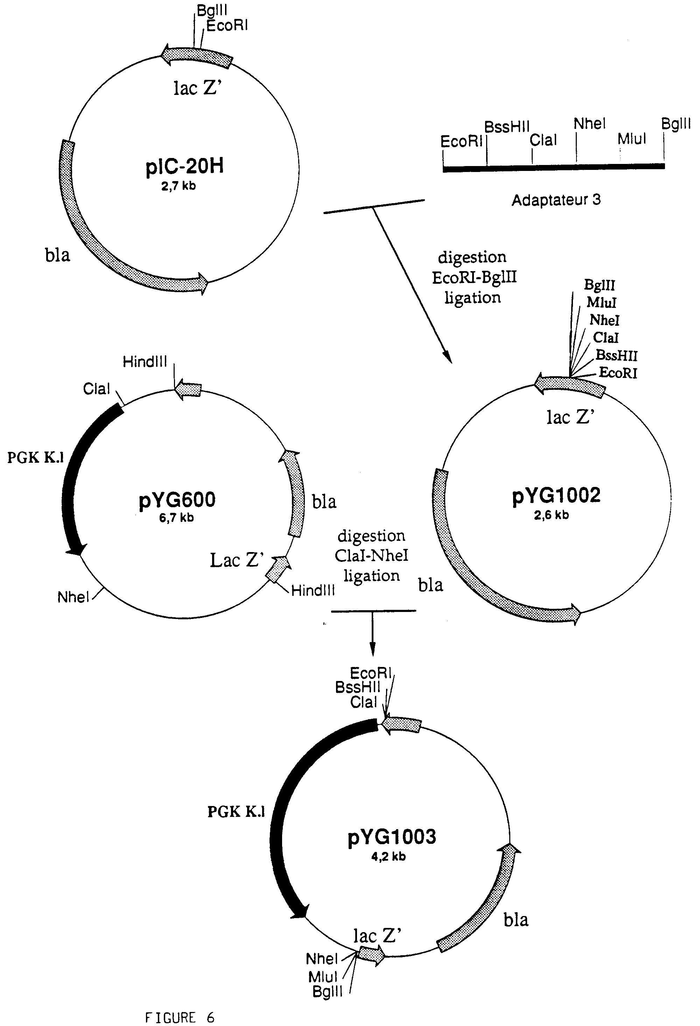

Le plasmide pYG1003 a été obtenu de la manière suivante (figure 6) :

Le plasmide pIC20H (Marsh et al., Gene 32 (1984) 481) a été digéré par BglII et EcoRI de façon à introduire l'adaptateur 3. Cet adaptateur, qui apporte les sites EcoRI, BssHII, ClaI, NheI, MluI et BglII a été obtenu comme décrit précédemment (2.2. (ii)), par hybridation des oligodésoxynucléotides E et F (figure 4). Le plasmide résultant est appelé pYG1002. Le gène PGK de K.lactis a été introduit dans ce nouveau plasmide sous forme d'un fragment ClaI-NheI, issu du plasmide pYG600. Le plasmide obtenu est appelé pYG1003 (figure 6).

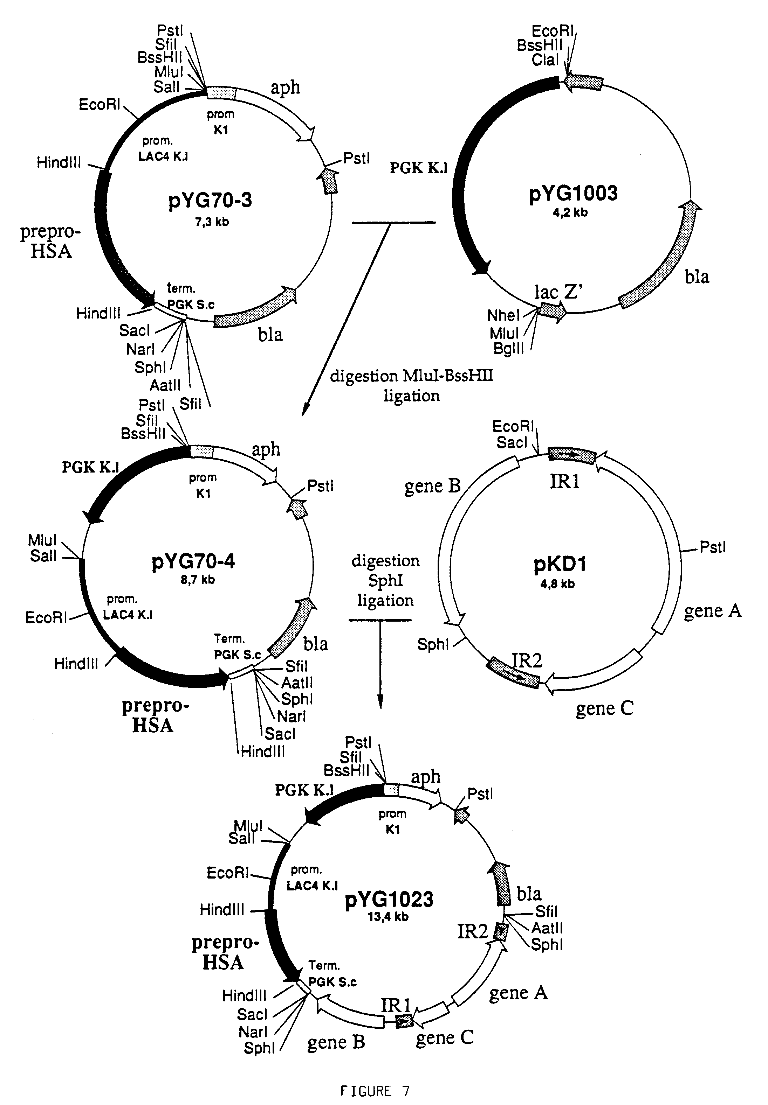

Le fragment MluI-BssHII issu du plasmide pYG1003 portant le gène PGK de K.lactis a ensuite été introduit dans les sites correspondants sur le plasmide pYG70-3, pour générer le plasmide pYG70-4 (figure 7).

Sur ce plasmide, le gène PGK de K.lactis est désormais placé sous le contrôle du promoteur bidirectionnel k1 de la toxine Killer.

### 2.5. Insertion du réplicon levure.

Les plasmides pYG70-4 (figure 7) et pKD1 (EP 361 991) ont été digérés par SphI et ligués ensemble en présence de ligase. A l'issue de cette ligation, 4 vecteurs peuvent être obtenus, selon la conformation du plasmide pKD1 (forme A ou forme B) et l'orientation de la partie correspondant au plasmide pYG70-4 par rapport à pKD1.

L'une de ces constructions à été sélectionnée et appelé pYG1023 (figure 7). Ce vecteur comprend :
- la séquence entière du plasmide pKD 1, qui fait de pYG1023 un plasmide à copies multiples, stable, et capable de se répliquer chez les levures et préférentiellement les levures du genre Kluyveromyces,
- une cassette d'expression de la sérum-albumine humaine comportant le gène de structure codant pour la forme prépro sous contrôle du promoteur inductible du gène LAC4 de K.lactis, et du terminateur du gène PGK de S.cerevisiae,
- un réplicon et un marqueur de sélection (gène bla conférant la résistance à l'ampicilline) pour E.coli,
- deux marqueurs de sélection pour la souche K.lactis FB05D (Cf exemple 3) : le gène aph muté sous contrôle du promoteur bidirectionnel k1 de la toxine Killer et le gène PGK de K.lactis sous contrôle du même promoteur mais transcrit de façon divergente par rapport au gène aph.

### 2.6. Construction du vecteur pYG1033ΔSfiI.

Une construction dérivée du plasmide pYG1023 a été réalisée, dans le but d'obtenir, une fois introduite dans la levure, des vecteurs d'expression dépourvus de réplicon bactérien et des marqueurs de résistance à l'ampicilline et à la généticine, et sur lesquels le promoteur K1 situé en amont du gène PGK est éliminé. Cette construction a été réalisée de la macère suivante :

### (i) Modification du terminateur PGK.

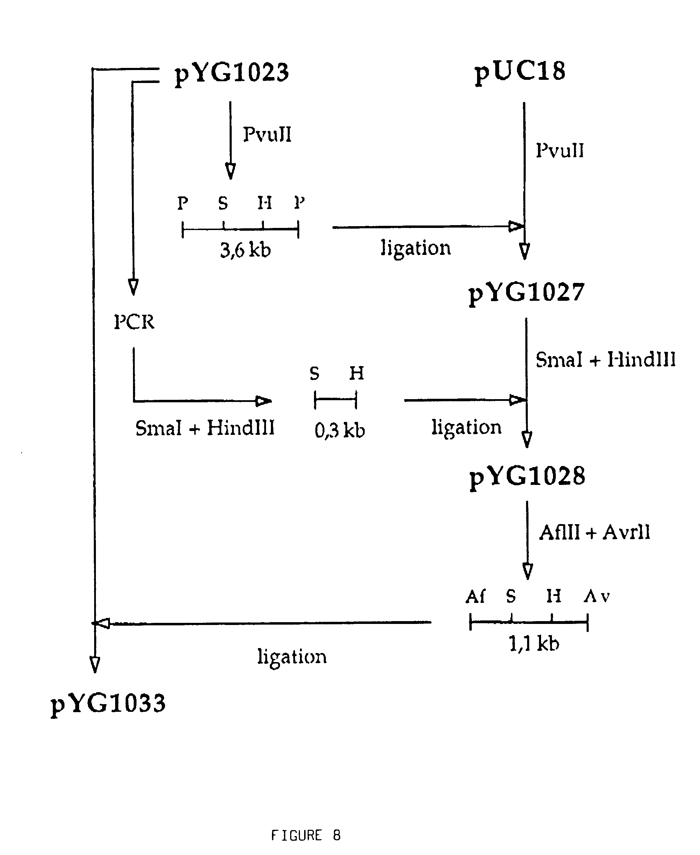

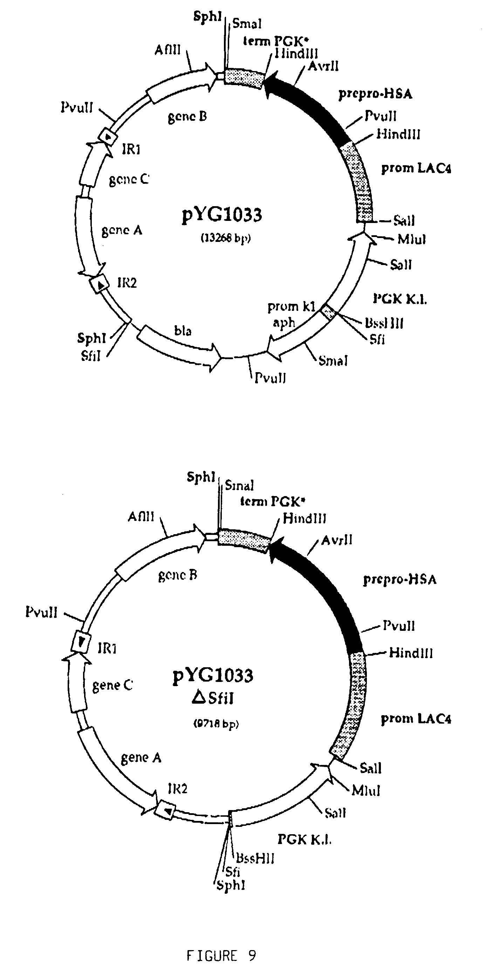

Cette étape est facultative. Toutefois elle a été réalisée, dans le but d'éviter tout risque de recombinaison au sein même du vecteur d'expression, qui conduirait à la diminution des capacités productrices du couple hôte/vecteur. En effet, le fragment utilisé comme terminateur dans le plasmide pYG1023 contient le terminateur PGK de S.cerevisiae et également la partie C-terminale du gène de structure PGK de S.cerevisiae, et présente de ce fait une homologie avec la région correspondante du gène PGK de K.lactis utilisé comme marqueur de sélection. Ce terminateur a donc été modifié de la manière suivante :
Le fragment PvuII de 3,6 kb isolé à partir de pYG1023 a dans un premier temps été sous cloné dans les sites correspondants du vecteur pUC18 pour former le plasmide pYG1027. Le fragment SmaI-HindIII de 427 pb de ce plasmide portant la région terminateur PGK a ensuite été remplacé par un fragment de 325 pb correspondant à la région 3′ non codante du gène PGK de S.cerevisiae. (c'est-à-dire ne contenant aucun element du gène de structure). Ce fragment de 325 pb a été obtenu par réaction d'amplification PCR (Cf techniques générales de clonage) sur le terminateur PGK présent sur le plasmide pYG1023 en utilisant les oligodésoxynucléotides suivants : 
5'-GGAAAGCTTCGGACCGTAAATTGAATTGAATTGAAATC-3'
5'-CCATCCCGGGAGCTCATCCGAATTAATTCCCAGC-3'
puis digéré avec les enzymes SmaI et HindIII. Le vecteur obtenu a été désigné pYG1028 (figure 8). La digestion de celui-ci avec les enzymes AvrII et AfII permet l'isolement d'un fragment de 1,1 kb, qui a été utilisé pour remplacer le fragment correspondant dans pYG1023. Le plasmide obtenu a été désigné pYG1033 (figure 9).

### (ii) Délétion du fragment SfiI.

Le plasmide pYG1033 a ensuite été soumis à une digestion en présence de l'enzyme SfiI pour exciser le fragment de 3,5 kb contenant le réplicon bactérien et les marqueurs de résistance, puis à une ligation en présence de ligase pour générer le plasmide pYG1033ΔSfiI (figure 9).

### 3. Isolement d'un mutant pgk de K.lactis.

Cet exemple décrit la préparation d'un mutant pgk à partir d'une souche sauvage de K.lactis en évitant l'utilisation de gènes de résistance à des antibiotiques. Deux étapes ont été effectuées successivement:
(i) préparation d'une souche auxotrophe ura3 (exemple 3.1.), et
(ii) remplacement du gène PGK ("gene replacement", Rothstein précitée) par un fragment d'ADN portant le gène PGK modifié par substitution d'une partie interne du gène par un fragment d'ADN portant le gène URA3 de S.cerevisiae. Le mutant ppk a ainsi été préparé par élimination dirigée de la quasi totalité de la phase ouverte de lecture (ORF) du gène PGK dans le génome de la souche ura3 (Cf exemple 3.2.).

Cette technique de mutagénèse permet d'éviter l'emploi d'agents mutagéniques non spécifiques, qui pourraient altérer d'autres régions du génome de la cellule. Elle permet également d'éviter tout événement génétique de réversion risquant de corriger les modifications effectuées sur le gène PGK,

### 3.1. Construction d'un mutant ura3 de K.lactis.

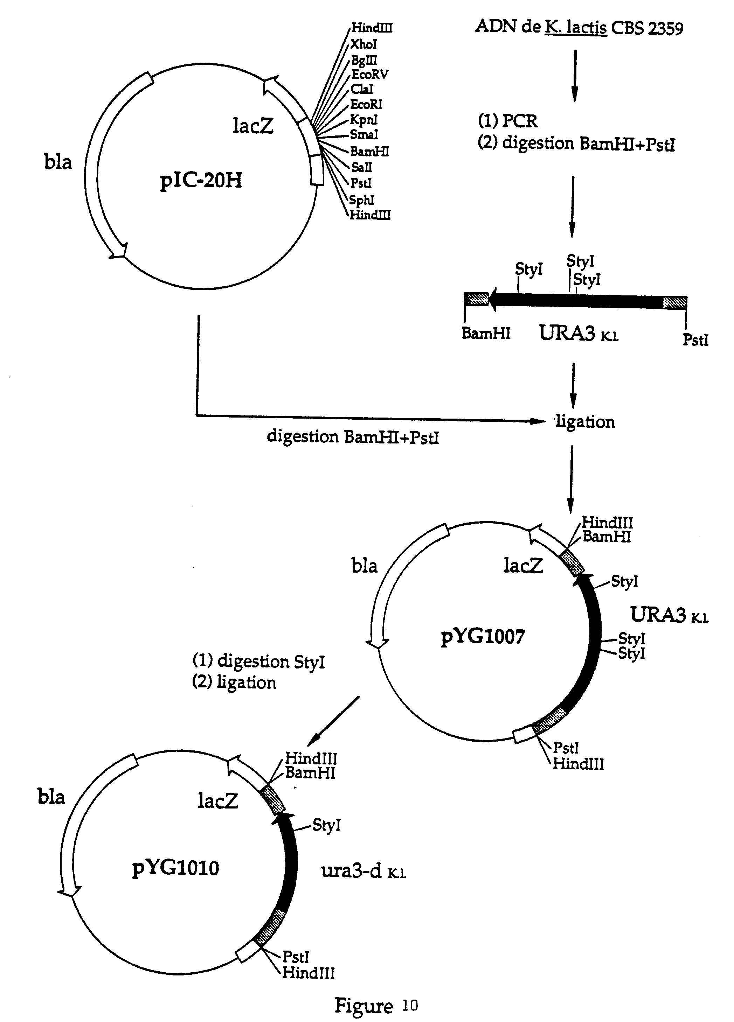

### (i) Clonage et modification du gène URA3 de K.lactis CBS2359 (figure 10).

Le gène URA3 de K.lactis codant pour l'orotidine-5-phosphate décarboxylase (Shuster et al., Nucl. Acid. Res. 15 (1987) 8573) a été cloné sous forme d'un fragment BamHI-PstI de 1,2 kb en utilisant la technique de PCR (Cf techniques générales de clonage), à partir d'un extrait d'ADN génomique (Rose et al., "Methods in Yeast Genetics" Cold Spring Harbor Laboratory Press, N.Y., 1990) de K.lactis CBS2359 au moyen des oligodéoxynucléotides suivants:

5'-GGAAGCTTGGCTGCAGGAATTGTCGTTCATGGTGACAC-3' et
5'-CCGAATTCCCGGATCCCATAATGAAAGAGAGAGAGAGAAGCAAAC-3'.

Le fragment obtenu a ensuite été sous-cloné dans les sites BamHI et PstI du plasmide pIC20H pour donner le plasmide pYG1007 (figure 10). Le gène URA3 a ensuite été modifié par délétion d'un fragment StyI interne à l'ORF comprenant 286 pb. Ceci a été effectué sur pYG1007, par digestion avec l'enzyme StyI puis ligation en présence de ligase. Ce nouveau plasmide est appelé pYG1010 (figure 10).

### (ii) Tranformation de K.lactis CBS 293.91 par le gène URA3 délété.

La souche CBS 293.91 a été transformée selon le protocole décrit par Durrens et al. (Curr. Genet. 18 (1990) 7) par 10 µg du fragment PstI-BamHI isolé par électroélution du plasmide pYG1010, qui contient le gène URA3 délété. Après un saut de température à 42°C ("heat shock") et 2 lavages successifs à l'eau, 600 µl de milieu YPD (extrait de levure 10 g/l ; peptone 20 g/l ; glucose 20 g/l) ont été ajoutés et les cellules ont été incubées une nuit. Les cellules ont ensuite été étalées sur milieu synthétique minimal SD ("bacto-yeast nitrogén base" sans acides aminés (Difco) 6,7 g ; glucose 20 g ; Bacto-agar 20 g, eau distillée 1000 ml) en présence d'uracile (100 µg/ml), d'uridine (100 µg/ml) et de 5-fluoro orotate (5FO) 15 mM. Des clones sont apparus au bout de 4 à 5 jours. Ils ont été repiqués sur milieu YPD de manière à obtenir des colonies isolées.

A partir du 1er repiquage, 3 clones issus de la colonie initialement apparue sur milieu SD+5FO ont été réisolés sur milieu YPD (repiquage secondaire).

Les clones issus du repiquage secondaire ont ensuite été testés pour le phénotype Ura3⁻ en utilisant le test en goutte sur milieu SD et SD+uracile (Jund et Lacroute, J. of Bact. 102 (1970) 607-615 ; Bach et Lacroute, Mol. Gen. Genet. 115 (1972) 126-130). Le génotype ura3 des clones ainsi obtenus a été contrôlé par:
- réaction de PCR en utilisant les oligodéoxynucléotides décrits pour le clonage en 4.1., ce qui permet d'identifier les clones portant le gène URA3, délété ou intact par différence de taille du produit d'amplification (0,9 et 1,2 kb respectivement);
- complémentation au moyen du plasmide pKan707 (EP 361 991) portant le gène URA3 intact de S.cerevisiae, connu pour sa capacité à complémenter la mutation ura3 chez K.lactis (De Louvencourt précitée) ; et

- Southern blot sur l'ADN génomique des clones identifés, en utilisant comme sonde le gène URA3 de K.lactis isolé dans l'exemple 4,1, marqué au ³²P selon la technique décrite par Feinberg et Vogelstein (Anal, Biochem, 132 (1983) 6). Cette étape permet d'identifier les clones portant le gène URA3 délété ou intact, par différence de taille du fragment révélé par hybridation.

Le mutant ura3 sélectionné est appelé K.lactis Y616.

### 3.2. Construction d'un mutant pgk de K.lactis Y616.

Un fragment d'ADN a été préparé, contenant le gène PGK modifié par substitution d'une partie interne du gène par un fragment d'ADN portant le gène URA3 de S.cerevisiae. Ce fragment a ensuite été utilisé pour remplacer la copie génomique intacte du gène PGK, par double recombinaison homologue (Rothstein précitée).

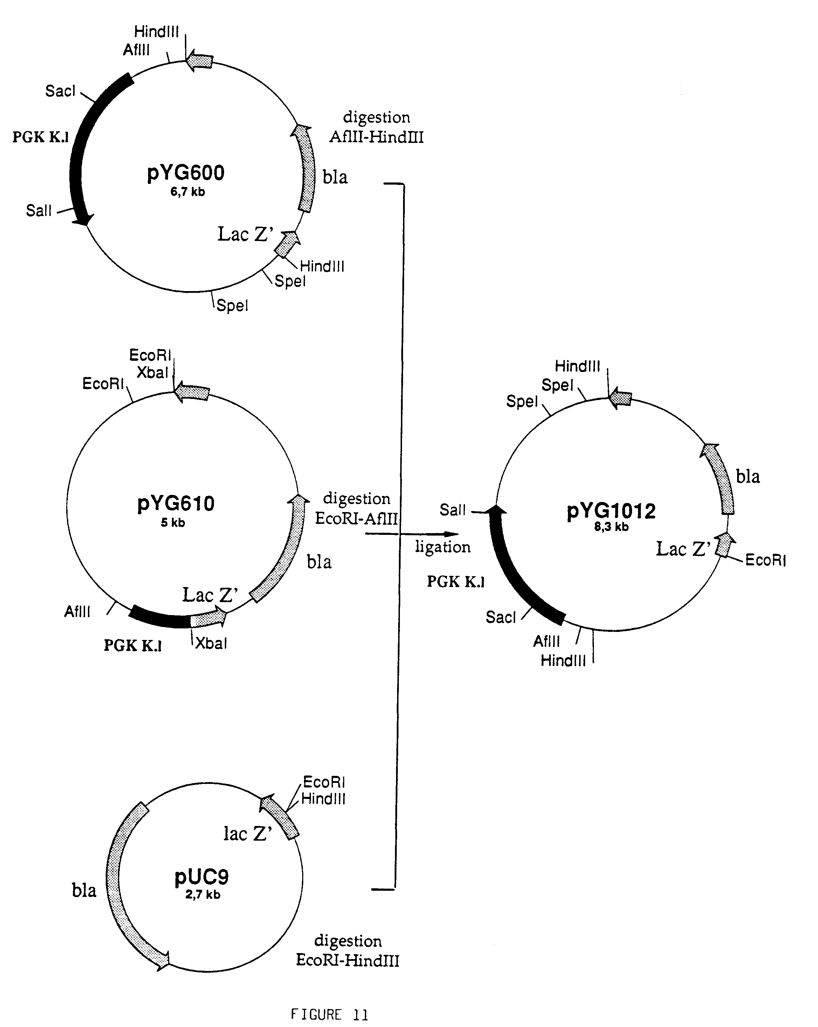

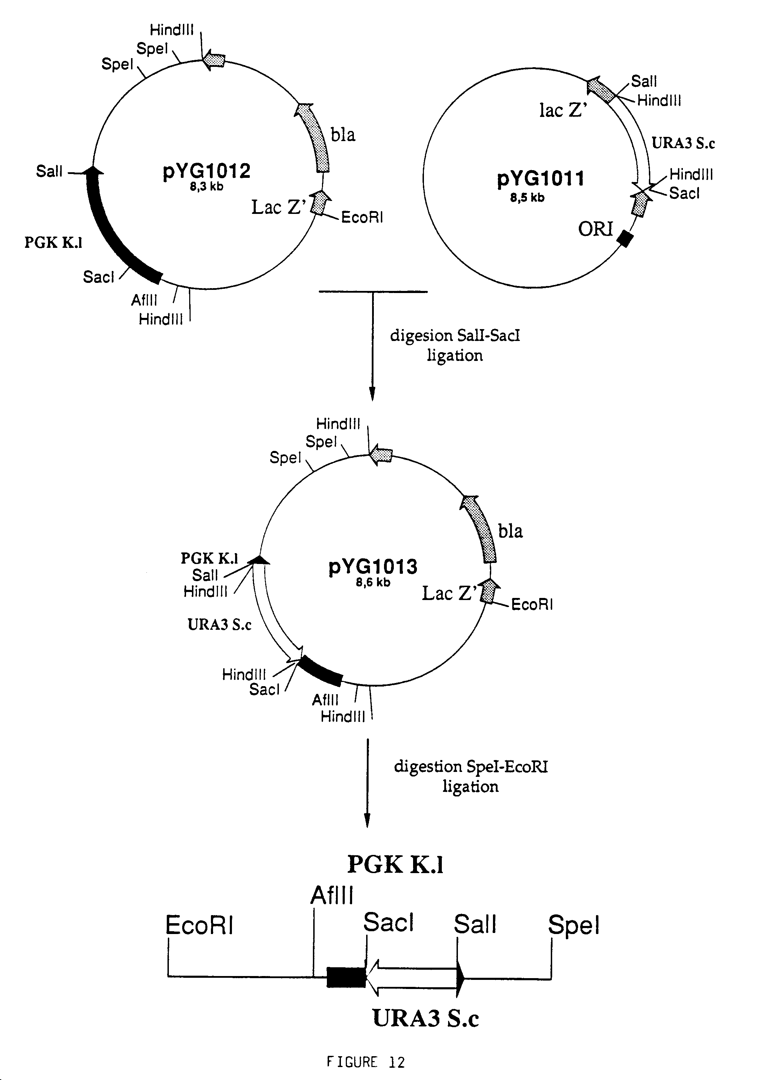

### (i) Construction d'un fragment d'ADN contenant le gène PGK modifié (figures 11 et 12).

Dans le but d'augmenter la fréquence de recombinaison homologue, le gène PGK a dans un premier temps été reconstitué bordé de régions flanquantes plus importantes. En particulier, les régions situées en amont du gène PGK ont été clonées, puis ligaturées au fragment porté par le plasmide pYG600 (figure 1).
- Clonage de la région en amont du gène PGK.
   Le criblage de la banque décrite à l'exemple 1 a également permis de mettre en évidence par "Southern blot" un fragment génomique XbaI de 2,5 kb environ. Ce fragment a été isolé par criblage d'une banque génomique restreinte de K.lactis CBS2359, constituée de fragments d'ADN coupés par XbaI, d'une taille comprise entre 2 et 3 kb, introduits dans le site XbaI du plasmide pUC18. Une banque de 500 clones a ainsi été constituée, puis criblée avec la sonde hétérologue utilisée dans l'exemple 1. Par hybridation sur colonies, un clone a été identifié et son ADN plasmidique séparé. Ce plasmide, nommé pYG610 (figure 11), contient un fragment d'ADN génomique de 2,5 kb. L'analyse de la séquence de ce fragment montre qu'il contient une partie codant pour la région N-terminale de la protéine Pgk de K.lactis (0,3 kb), et 2,2 kb correspondant à la région située en amont du gène PGK.
- Construction du plasmide pYG 1012 (figure 11 ).
   Les fragments AflII-HindIII du plasmide pYG600 et EcoRI-AfII du plasmide pYG610 ont été isolés par électroélution, puis introduits ensemble par ligation dans le plasmide pUC9 préalablement digéré par EcoRI et HindIII. Le plasmide obtenu est appelé pYG1012.
- Modification du gène PGK.

Le fragment SalI-SacI interne au gène PGK porté par le plasmide pYG1012 a été remplacé par digestion puis ligation par le fragment SalI-SacI issu du plasmide pYG1011, portant le gène URA3 de S.cerevisiae, pour générer le plasmide pYG1013 (figure 12).

Le plasmide pYG1011 a été construit par insertion d'un fragment HindIII isolé à partir du plasmide YEp24 (ATCC n° 37051) contenant le gène URA3 de S.cerevisiae, dans les sites correspondants du bactériophage M13tg130 (Kieny et al., Gene 26 (1983) 101).

Le plasmide pYG1013 contient donc, sous forme d'un fragment EcoRI-SpeI, le gène URA3 de S.cerevisiae, bordé d'un coté par la région 5′ non codante et les 500 premières paires de bases du gène de structure PGK de K.lactis, et de l'autre par les 100 dernières paires de bases du gène de structure PGK de K.lactis et son terminateur (figure 12).

### (ii) Transformation de K.lactis Y616 par le gène PGK modifié..

La souche Y616 (ura3) a été transformée selon la méthode décrite par Durrens et al (précitée), avec 10 µg du fragment EcoRI-SpeI isolé à partir du plasmide pYG1013.

Après transformation, les cellules contenant le gène URA3 fonctionnel ont été sélectionnées sur milieu synthétique minimal SD dans lequel le glucose a été remplacé du glycérol (3 %) et de l'éthanol (2 %). Ce milieu permet la croissance des mutants pgk.

Les colonies transformées ainsi obtenues ont ensuite été repiquées sur milieu complexe YPD, dans lequel les mutants pgk sont incapables de pousser. 50 % des souches obtenues ont présenté dans ce test un phénotype Pgk⁻.

Sur les colonies ainsi identifiées, la mutation au niveau du gène PGK a été contrôlée par réaction d'amplification PCR en utilisant les oligodésoxynucléotides G et H décrits sur la figure 4. Ces 2 oligodésoxynucléotides permettent d'amplifier:
- une région de 0,9 kb environ sur le gène PGK de K.lactis intact, et
- une région de 1,3 kb environ sur le gène PGK de K.lactis modifié comme décrit ci-dessus (i).

L'amplification a été réalisée sur cellules entières présentant le phénotype Pgk⁻. Après 30 cycles d'amplification, les surnageants (10 µl) ont été déposés sur gel d'agarose (0,8 %) afin de déterminer la taille des bandes. Les témoins ont été constitués par amplification au moyen des mêmes oligodésoxynucléotides sur les plasmides pYG600 gène intact) et pYG1013 (gène modifié).

Les résultats obtenus montrent que dans les souches Pgk-, une bande de 1,3 kb est amplifiée. Ces souches possèdent donc bien un gène PGK modifié. Parmi celles-ci, un mutant pgk a été sélectionné et appelé K.lactis FB05D.

### 4. Transformation de la souche K.lactis FB05D par les plasmides pYG1023 et pYG1033ΔSfiI.

Le vecteur pYG1023 a été introduit par transformation dans la souche K.lactis FB05D en utilisant la technique éthylène glycol/diméthylsulfoxyde (Dwrens et al précitée). Les levures transformées ont été sélectionnées pour le phénotype Pgk⁺ que confère le plasmide pYG1023 sur milieu complexe YPD.

Le vecteur pYG1033ΔSfiI a été introduit dans la souche K.lactis FB05D par électroporation, selon la technique décrite par Meilhoc et al. (Biotechnologie 8 (1990) 223). Après transformation, les cellules ont été étalées sur milieu YPD et cultivées à 30°C pendant 3 jours. Les cellules capables de croitre sur ce type de milieu (contenant donc une copie fonctionnelle du gène PGK) ont ensuite été testées pour leur résistance à la généticine. 60 % d'entre-elles sont incapables de pousser sur milieu YPD en présence de 200 µg/ml de G418. Ces résultats montrent que le vecteur introduit a perdu le marqueur G418, et qu'il est capable de complémenter la mutation pgk de la souche FB05D.

### 5. Etude de la stabilité du plasmide pYG1023 et de la production d'albumine.

### 5. 1. Etude de stabilité.

Les cellules transformées ont été précultivées en erlenmeyers dans un milieu complexe de type industriel M9CS20 [Milieu M9 (Maniatis et al. précitée) supplémenté de 20 g/l d'extrait soluble de maïs (Solulys.L, Roquette) en présence d'acétate d'ammonium 2 g/l et de lactose 20 g/l] à 28°C sous agitation.

Cette préculture a ensuite été utilisée pour inoculer, à une dilution de 10⁻³ (erlen 1) et de 10⁻⁶ (erlen 2), 2 erlenmeyers de 300 ml contenant 50 ml de milieu M9CS20. Les cellules ont ensuite été cultivées comme précédemment sous agitation (200 tours/min) pendant 3 jours. A ce stade, les cellules de l'erlen 1 ont subi 10 divisions cellulaires (temps 10 générations), et les cellules de l'erlen 2 ont subi 20 divisions cellulaires (temps 20 générations).

La culture de l'erlen 2 a ensuite été utilisée à son tour pour inoculer, à une dilution de 10⁻³ (erlen 3) et de 10⁻⁶ (erlen 4), 2 autres erlenmeyers. Les cellules ont ensuite été cultivées comme précédemment pendant 3 jours. A ce stade, les cellules de l'erlen 3 ont subi 30 divisions cellulaires (temps 30 générations), et les cellules de l'erlen 4 ont subi 40 divisions cellulaires (temps 40 générations).

Cette opération a été répétée encore 8 fois, de manière à obtenir les erlens 5-20 contenant des cultures ayant subi de 50 jusqu'à 200 divisions cellulaires.

A la fin de chaque culture, un échantillon a été prélevé dans les erlenmeyers, de manière à réaliser une suspension à 10³ cellules/ml. 200 µl de ces suspensions ont ensuite été étalés sur 2 types de boites :
- boîte YPGE (extrait de levure 10 g/l, peptone 20 g/l, glycérol 30 g/l, éthanol 20 g/l). Ce milieu permet la croissance de toutes les cellules.
- boîte YPD en présence de G418 (200 µg/ml). Sur ce type de boite, seules les cellules contenant le plasmide pYG1023 portant le gène de résistance au G418 et le gène PGK sont capables de pousser.

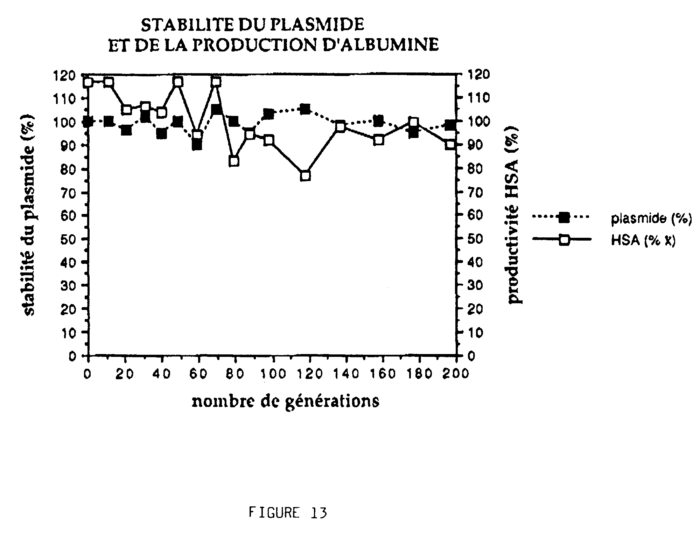

La stabilité du plasmide pYG1023 a ainsi été déterminée pour chaque culture. Les résultats sont présentés sur la figure 13, dans laquelle la stabilité est définie par le rapport du nombre de colonies présentes sur les boites YPD/G418 sur le nombre de colonies présentes sur les boites YPGE.

Ces résultats démontrent les avantages de l'invention par rapport à l'art antérieur. En effet, bien que des plasmides stables basés sur pKD1 aient déjà été décrits, l'introduction de cassettes d'expression dans ceux-ci permettant la production de protéines recombinantes s'est toujours accompagnée d'une baisse de stabilité. Ceci a en particulier été observé pour l'albumine et l'interleukine-1β (EP 361 991). La présente invention permet d'éliminer cette perte de stabilité, puisque, dans des conditions d'induction de l'expression (lactose), les vecteurs sont maintenus dans 100 % des cellules transformées après 200 générations de culture.

### 5.2. Etude de la production d'albumine.

A la fin de chaque culture obtenue selon la procédure décrite en 5.1., 10 µl de surnageant dépourvu de cellules ont été prélevés dans chaque erlen, et mélangés à un volume équivalent de tampon Laemmli 2X (Laemmli, Nature 227 (1970) 680). Après chauffage à 96°C pendant 10 minutes, les protéines de l'échantillon ont été séparées sur gel de polyacrylamide SDS 8,5 %. La production d'albumine a ensuite été révélée par coloration du gel au bleu de Coomassie, et évaluée par densitométrie en utilisant un densitomètre Shimazu CS930 (marge d'imprécision de cette technique : +/- 20 %). La figure 13 montre la variation de la production d'albumine en fonction du nombre de générations de culture en condition d'induction (milieu contenant du lactose). Les valeurs sont données en pourcentage de production par rapport à la production moyenne mesurée sur les 200 générations.

Ces résultats confirment la stabilité importante du couple hôte vecteur décrit, et sa capacité à produire à des niveaux constants et élevés, en condition d'induction, une protéine recombinante sur 200 générations de culture au moins en milieu complexe de type industriel.

Un échantillon des souches K.lactis Y616 et K.lactis FB05D a été déposé le 11 juin 1991 auprès du Centraalbureau voor Schimmelkulturen (CBS) à Baarn aux Pays-Bas dans les conditions du Traité de Budapest, sous les numéros respectifs CBS 294.91 et CBS 295.91. La souche K.lactis CBS 293.91 correspond à la souche CBS1065 redéposée le 11 juin 1991 selon les conditions du Traité de Budapest.

## Revendications

1. Couple hôte/vecteur hautement stable en milieu complexe caractérisé en ce que l'hôte est une levure du genre Kluyveromyces dans laquelle un gène essentiel pour la croissance dans ledit milieu est non-fonctionnel, et en ce que le vecteur porte une copie fonctionnelle dudit gène.

2. Couple hôte/vecteur selon la revendication 1 caractérisé en ce que le gène essentiel est choisi parmi les gènes impliqués dans le métabolisme d'une source carbonée présente dans le milieu (galactose, lactose, glucose, etc), les gènes intervenant dans la division cellulaire, dans la synthèse membranaire, dans la synthèse protéique, dans la réplication ou la transcription de l'ADN.

3. Couple hôte/vecteur selon la revendication 2 caractérisé en ce que le gène essentiel est un gène impliqué dans la glycolyse.

4. Couple hôte/vecteur selon la revendication 3 caractérisé en ce que le gène impliqué dans la glycolyse est un gène unique dont le produit est indispensable pour que la levure puisse métaboliser les sources de carbone du milieu.

5. Couple hôte/vecteur selon la revendication 4 caractérisé en ce que le gène impliqué dans la glycolyse est choisi parmi les gènes codant pour la phosphoglycérate kinase (PGK), la phosphoglycérate musse (GPM), la pyruvate kinase (PYK), et la triose phosphate isomérase (TPI).

6. Couple hôte/vecteur selon la revendication 1 caractérisé en ce que la copie fonctionnelle du gène essentiel présente sur le vecteur est soit placée sous le contrôle d'un promoteur faible, soit entièrement dépourvue de promoteur, soit placée sous le contrôle d'un promoteur défectif.

7. Couple hôte/vecteur selon la revendication 1 caractérisé en ce que le gène essentiel présent sur le vecteur est défectif dans certaines conditions, comme par exemple de température gène thermosensible).

8. Couple hôte/vecteur selon l'une quelconque des revendications 1 à 7 caractérisé en ce que l'hôte est choisi parmi les levures Kluyveromyces lactis et Kluyveromyces fragilis.

9. Couple hôte/vecteur selon la revendication 1 caractérisé en ce que le vecteur comprend en outre une sequence d'ADN comportant un gène de structure au moins codant pour une protéine recherchée et des signaux permettant son expression.

10. Couple hôte/vecteur selon la revendication 9 caractérisé en ce que le gène de structure code pour une protéine d'intérêt pharmaceutique ou agroalimentaire.

11. Couple hôte/vecteur selon la revendication 10 caractérisé en ce que le gène de structure code pour une protéine choisie parmi les enzymes (tels que notamment la superoxide dismutase, la catalase, les amylases, les lipases, les amidases, la chymosine), les dérivés sanguins (tels que la sérum-albumine ou des variants de celle-ci, l'alpha- ou la béta-globine, le facteur VIII, le facteur IX, le facteur von Willebrand ou des domaines de celui-ci, la fibronectine, l'alpha-1 antitrypsine), l'insuline et ses variants, les lymphokines (telles que les interleukines, les interférons, les facteurs de stimulation des colonies (G-CSF, GM-CSF, M-CSF), le TNF, le TRF, les MIP), les facteurs de croissance (tels que l'hormone de croissance, l'érythropoiétine, le FGF, l'EGF, le PDGF, le TGF), les apolipoprotéines, des polypeptides antigéniques pour la réalisation de vaccins (hépatite, cytomégalovirus, Eppstein-Barr, herpes etc.), des récepteurs viraux, ou encore des fusions de polypeptides telles que notamment des fusions comportant une partie active fusionnée à une partie stabilisatrice.

12. Couple hôte/vecteur selon la revendication 9 caractérisé en ce que la séquence d'ADN comprend en outre des signaux permettant la sécrétion de la protéine recombinante.

13. Couple hôte/vecteur selon la revendication 9 caractérisé en ce que les signaux permettant l'expression du gène de structure sont choisis parmi les promoteurs et les terminateurs de transcription.

14. Couple hôte/vecteur selon la revendication 13 caractérisé en ce que les promoteurs utilisés sont des promoteurs de gènes de levure, éventuellement modifiés, et encore plus préférentiellement, de gènes glycolytiques de levure ou de gènes de levure fortement exprimés.

15. Procédé de production d'une protéine recombinante caractérisé en ce que l'on cultive un couple hôte/vecteur tel que défini dans les revendications 9 à 14, et on récupère la protéine produite.

16. Procédé selon la revendication 15 pour la production de protéines d'intérêt pharmaceutique ou agroalimentaire.

17. Procédé selon la revendication 16 pour la production de protéines telles que définies dans la revendication 11.

18. Procédé selon la revendication 17 pour la production de sérum-albumine humaine, ses précurseurs et variants moléculaires.

19. Utilisation d'un couple hôte/vecteur tel que défini dans les revendications 9 à 14 comme catalyseur dans des réactions de bioconversion.

20. Vecteur d'expression pour les levures du genre Kluyveromyces portant une copie fonctionnelle d'un gène essentiel à la croissance de la levure Kluyveromyces sur milieu complexe.

21. Vecteur selon la revendication 20 caractérisé en ce que le gène essentiel est défini selon l'une des revendications 2 à 5.

22. Vecteur selon la revendication 20 caractérisé en ce qu'il est dépourvu de séquences bactériennes.

23. Les vecteurs pYG1023, pYG1033 et pYG1033ΔSfiI, tels que décrits sur les figures 7 et 9.

24. Levure du genre Kluyveromyces présentant le phénotype Pgk**⁻.**

25. La levure K.lactis FB05D n° CBS 295.91.


LISTE DE SEQUENCES

(1) INFORMATION GENERALE:

(i) DEPOSANT:
(A) NOM: RHONE-POULENC RORER S.A.
(B) RUE: 20 avenue Raymond ARON
(C) VILLE: ANTONY
(E) PAYS: FRANCE
(F) CODE POSTAL: 92165

(ii) TITRE DE L' INVENTION: LEVURES RECOMBINANTES HAUTEMENT
STABLES POUR LA PRODUCTION DE PROTEINES RECOMBINANTES, LEUR
PREPARATION ET LEUR UTILISATION.

(iii) NOMBRE DE SEQUENCES: 8

(iv) FORME LISIBLE PAR ORDINATEUR:
(A) TYPE DE SUPPORT: Tape
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(v) DONNEES DE LA DEMANDE ACTUELLE:
NUMERO DE DEPOT: EP 92402212.2
(vi) DONNEES DE LA DEMANDE ANTERIEURE:
(A) NUMERO DE DEPOT: FR FR 91 09854
(B) DATE DE DEPOT: 02-AUG-1991

(2) INFORMATION POUR LA SEQ ID NO: 1:

(i) CARACTERISTIQUES DE LA SEQUENCE:
(A) LONGUEUR: 37 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:

(B) CLONE: Adaptateur 1 - Oligonucleotide A

(xi) DESCRIPTION DE LA SEQUENCE: SEQ ID NO: 1:

CGTCGACACG CGTGCGCGCC CGCGGCCAAT GGGGCCC 37

(2) INFORMATION POUR LA SEQ ID NO: 2:

(i) CARACTERISTIQUES DE LA SEQUENCE:
(A) LONGUEUR: 45 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: Adaptateur 1 - Oligonucleotide B

(xi) DESCRIPTION DE LA SEQUENCE: SEQ ID NO: 2:

TCGAGGGCCC CATTGGCCGC GGGCGCGCAC GCGTGTCGAC GAGCT 45

(2) INFORMATION POUR LA SEQ ID NO: 3:

(i) CARACTERISTIQUES DE LA SEQUENCE:
(A) LONGUEUR: 41 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: Adaptateur 2 - Oligonucleotide C

(xi) DESCRIPTION DE LA SEQUENCE: SEQ ID NO: 3:

AATTAGGCCA ATGGGGCCGA CGTCGCATGC GGCGCCGAGC T 41

(2) INFORMATION POUR LA SEQ ID NO: 4:

(i) CARACTERISTIQUES DE LA SEQUENCE:
(A) LONGUEUR: 33 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: Adaptateur 2 - Oligonucleotide D

(xi) DESCRIPTION DE LA SEQUENCE: SEQ ID NO: 4:

CGGCGCCGCA TGCGACGTCG GCCCCATTGG CCT 33

(2) INFORMATION POUR LA SEQ ID NO: 5:

(i) CARACTERISTIQUES DE LA SEQUENCE:
(A) LONGUEUR: 41 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: Adaptateur 3 - Oligonucleotide E

(xi) DESCRIPTION DE LA SEQUENCE: SEQ ID NO: 5:

AATTCCCCGC GCGCCCATCG ATCCGCTAGC CCACGCGTCC A 41

(2) INFORMATION POUR LA SEQ ID NO: 6:

(i) CARACTERISTIQUES DE LA SEQUENCE:

(A) LONGUEUR: 41 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: Adaptateur 3 - Oligonucleotide F

(xi) DESCRIPTION DE LA SEQUENCE: SEQ ID NO: 6:

GATCTGGACG CGTGGGCTAG CGGATCGATG GGCGCGCGGG G 41

(2) INFORMATION POUR LA SEQ ID NO: 7:

(i) CARACTERISTIQUES DE LA SEQUENCE:
(A) LONGUEUR: 19 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: PCR - Oligonucleotide G

(xi) DESCRIPTION DE LA SEQUENCE: SEQ ID NO: 7:

TGCCTTCGGT ACCGCTCAC 19

(2) INFORMATION POUR LA SEQ ID NO: 8:

(i) CARACTERISTIQUES DE LA SEQUENCE:
(A) LONGUEUR: 20 paires de bases
(B) TYPE: acide nucléique
(C) NOMBRE DE BRINS: simple
(D) CONFIGURATION: linéaire

(ii) TYPE DE MOLECULE: ADNc

(vii) SOURCE IMMEDIATE:
(B) CLONE: PCR - Oligonucleotide H

(xi) DESCRIPTION DE LA SEQUENCE: SEQ ID NO: 8:

AGAAGGGAAG GGATGATGGA 20